# EUROPÄISCHE PATENTSCHRIFT

(11) **EP 1 521 964 B1**
(45) Veröffentlichungstag und Bekanntmachung des Hinweises auf die Patenterteilung: **26.12.2012**
(21) Anmeldenummer: 03763669.3
(22) Anmeldetag: 30.06.2003
(51) Int. Cl.: G01N 33/543, G01N 33/566, G01N 33/551, G01N 33/542, G01N 33/58, C12Q 1/68, G01N 33/53

(54) **VERFAHREN ZUR BESTIMMUNG DER ZAHL VON REZEPTOREN AUF EINEM TRÄGER**
METHOD FOR DETERMINING THE NUMBER OF RECEPTORS ON A CARRIER
PROCEDE POUR DETERMINER LE NOMBRE DE RECEPTEURS QUI SE TROUVENT SUR UN SUPPORT

(30) Priorität: 12.07.2002 DE 10231684
(43) Veröffentlichungstag der Anmeldung: 13.04.2005
(73) Patentinhaber: Endress+Hauser Conducta Gesellschaft für Mess- und Regeltechnik mbH+Co. KG, 70839 Gerlingen (DE)
(72) Erfinder: KLAPPROTH, Holger, 79108 Freiburg (DE); SIEBEN, Ulrich, 79276 Reute (DE)
(74) Vertreter: Koslowski, Christine Adelheid
(86) Internationale Anmeldenummer: PCT/EP2003/006948
(87) Internationale Veröffentlichungsnummer: WO 2004/008143

(56) Entgegenhaltungen:
- WO-A-00/68692
- US-B1- 6 197 503
- PETER C ET AL: "OPTICAL DNA-SENSOR CHIP FOR REAL-TIME DETECTION OF HYBRIDIZATION EVENTS" FRESENIUS JOURNAL OF ANALYTICAL CHEMISTRY, SPRINGER, BERLIN, DE, Bd. 371, Nr. 2, September 2001 (2001-09), Seiten 120-127, XP009016890 ISSN: 0937-0633
- LEHR H-P ET AL: "REAL-TIME DETECTION OF NUCLEIC ACID INTERACTIONS BY TOTAL INTERNAL REFLECTION FLUORESCENCE" ANALYTICAL CHEMISTRY, AMERICAN CHEMICAL SOCIETY. COLUMBUS, US, Bd. 75, Nr. 10, 15. Mai 2003 (2003-05-15), Seiten 2414-2420, XP001170913 ISSN: 0003-2700

## Beschreibung

Die vorliegende Erfindung betrifft ein Verfahren zur Bestimmung der Zahl von Rezeptoren auf einem Träger sowie einen Biosensor, insbesondere einen Proteinsensor, der mit Hilfe des Verfahrens herstellbar ist.

Biologische Systeme beruhen auf der Interaktion von biologisch aktiven Makromolekülen, die andere Moleküle über ihre dreidimensionale Oberflächenstruktur sowie eine spezifische elektronische Ladungsverteilung in der Regel reversibel binden. Neben reversiblen Bindungen sind kovalente Bindungen zwischen Molekülen bekannt, die genutzt werden, um Moleküle mittels chemischer Methoden an Oberflächen zu binden. Moleküle, die Bindungsaffinitäten für andere Molekülen besitzen, werden zusammenfassend als Rezeptoren bezeichnet, die eine entscheidende Rolle bei der Wechselwirkung und dem Zusammenspiel biologischer Systeme spielen. Beispiele für in der Natur vorkommende Rezeptoren sind Enzyme, welche die Umsetzung eines bestimmten Substrates katalysieren, Proteine, die den Transport von geladenen Molekülen über eine Biomembran ermöglichen, durch Zucker modifizierte Proteine (= Glykoproteine), die den Kontakt zu anderen Zellen erlauben, Antikörper, die im Blut zirkulieren und Bestandteile von Krankheitserregern wie Bakterien oder Viren erkennen, binden und inaktivieren. Im Rahmen biologisch aktiver Systeme wird auch DNA, der Träger der Erbinformation als Rezeptor verstanden. DNA besteht grundsätzlich aus zwei zueinander komplementären Strängen, die über Basenpaarungen und Wasserstoffbrückenbindungen eine Doppelhelix bilden. Jeder einzelne DNA Strang wirkt dabei als Rezeptor für seinen komplementären DNA Strang, der seinerseits die Funktion des Liganden wahrnimmt.

Alle Moleküle, die von einem Rezeptor spezifisch gebunden werden, werden zusammenfassend als Liganden bezeichnet, wobei von vielen biologisch aktiven Molekülen bekannt ist, dass sie einerseits selbst andere Moleküle binden, anderseits aber auch von Molekülen gebunden werden. Sie sind daher, abhängig von ihrem jeweiligen Bindungspartner, sowohl Liganden als auch Rezeptoren.

Zur Untersuchung von Interaktionen zwischen Rezeptoren und Liganden wurden eine Vielzahl von Testsystemen (Assays) entwickelt, mit deren Hilfe die Konzentration des Liganden in einer Probenlösung qualitativ und/oder quantitativ bestimmt wird. Solche Testsysteme werden aufgrund der großen Spezifität von Rezeptor-Liganden-Komplexen in der Kriminalistik bei der Überprüfung Tatverdächtiger, beim Vaterschaftstest, in der Krebsvorsorge, in der pränatalen Diagnostik, bei der Erstellung von Stammbäumen in der Wissenschaft und Forschung sowie für die Überprüfung von erfolgreichen Schutzimpfungen verwendet.

Nachdem die vollständigen genomischen DNA-Sequenzen wichtiger Modell- und Forschungsorganismen wie Bakterien (Bacillus subtilis, Escherichia coli) und Hefen (Saccharomyces cerevisiae) bereits seit Jahren in Datenbanken vorliegen, wurde zwischenzeitlich auch die Sequenzierung des menschlichen Genoms im Rahmen des Humangenomprojektes abgeschlossen. Da die Zahl der identifizierten menschlichen Gene sehr viel geringer als vermutet ist, gewinnt die Erforschung der Funktion der einzelnen Gene, die in verschiedenen Geweben und Organen unterschiedlich aktiv sind, seither zunehmend an Bedeutung.

Neben der detaillierten Erforschung der DNA ist in der jüngeren Vergangenheit die Untersuchung des Proteinanteils der Zelle, die auch als Proteomanalyse bezeichnet wird, immer wichtiger geworden. Die meisten pharmazeutisch aktiven Stoffe, die als Arzneimittel eingesetzt werden, wirken über die Beeinflussung von Proteinen. Solche Interaktionen können durch DNA Analysen nicht oder nur unzureichend analysiert werden.

Die Aufklärung der differentiellen Genexpression gilt als entscheidend für das Verständnis der Entwicklung vieler Krankheiten. Seit vielen Jahren werden daher vielfältige Versuche unternommen, eine möglichst große Zahl von biologisch aktiven Molekülen auf kleinstem Raum künstlich zu synthetisieren und anzuordnen, um sie bezüglich ihrer Interaktion mit anderen Molekülen untersuchen zu können. Für den quantitativen und qualitativen Nachweis von Interaktionspartnern bzw. Liganden in einer zu analysierenden Probe, beispielsweise einer Speichel- oder Blutprobe, werden planare Systeme verwendet, die als Biochips bzw. Biosensoren bezeichnet werden. Die Biosensoren bilden einen Träger, auf dessen Oberfläche eine Vielzahl von rasterartig angeordneten Nachweisbereichen ausgebildet ist. Bei der Herstellung solcher Biosensoren wurden zunächst die einzelnen Monomere über Mikrodosierung auf die Vielzahl der Einzelbereiche des Rasters aufgetragen, an denen ein Polymer gebildet werden sollte. Dieses Verfahren ist für breit angelegte Screening-Studien nicht geeignet, so dass Systeme zur lichtgesteuerten Polymersynthese unter Verwendung individueller Maskensätze wie aus der Halbleiterindustrie bekannt, zur Herstellung von Biosensoren verwendet wurden (Pease et al.(1994), PNAS, USA, Vol. 91, S. 5022 - 5026).

Bei den bekannten Testsystemen, die zum Nachweis von Liganden in zu analysierenden Proben verwendet werden, spielt der Nachweis eines auf der Oberfläche eines Biosensors gebildeten Rezeptor-Liganden-Komplexes die entscheidende Rolle. Bei herkömmlichen Systemen muss für die Konzentrationsberechnung des Liganden eine Kalibrierungskurve erstellt werden, aus der sich indirekt die Zahl der Ligandenmoleküle bzw. deren Konzentration bestimmen lässt.

Besonders häufig werden auch Systeme verwendet, bei denen versucht wird, die Liganden in der zu analysierenden Probe selbst zu markieren. Daran ist insbesondere nachteilig, dass die Reaktion des Liganden mit einem Marker, beispielsweise einem Farbstoff zu einer Konfigurations- oder Konformations-änderung des Liganden und somit zu einer Veränderung seiner Oberflächenstruktur führen kann. Da aber gerade die dreidimensionale Oberfläche für die Bindung des Liganden an den auf der Oberfläche des Biosensors fixierten Rezeptor von entscheidender Bedeutung ist, liefert die direkte Markierung von Liganden in der Regel keine zufriedenstellenden Ergebnisse.

Als Marker wurden darüber hinaus molekulare Beacons entwickelt, die von Schonfield et al., (1997), Applied and Environmental Microbiology, Vol. 63, S. 1143 - 1147 sowie von Tyagi und Kramer (1996), Nature Biotech., Vol. 14, S. 303 - 308 beschrieben wurden. Molekulare Beacons sind DNA-Sonden, die eine kurze komplementäre Sequenz von Nukleotiden aufweisen, die an den 5'- und 3'-Enden der Probensequenz angeordnet sind, so dass sich eine Stammstruktur ("Stem-Loop-Struktur") in Lösung bildet. Ein Farbstoff, insbesondere ein Fluorochrom und ein geeigneter Dämpfer ("Quencher") sind über Linker an den Enden des Stem-Loops angeordnet. Diese Stem-Loop-Struktur bildet den Rezeptor, in dem in Abwesenheit eines Liganden das Fluorochrom und der Quencher über die Stammstruktur nahe beieinander gehalten werden, so dass die Fluoreszenz unterdrückt ist. Wenn jedoch der einzelsträngige Loop mit einer komplementären Zielsequenz (= Ligand) interagiert und stabil hybridisiert, denaturiert die Stem-Loop-Struktur. Dadurch tritt Fluoreszenz auf, weil sich ein stabilerer Hybrid aus Loop und Zielsequenz (= Rezeptor-Liganden-Komplex) ausbildet, der nicht mit der weniger stabilen internen Basenpaarung des Stammhybriden koexistieren kann. Da diese Sonden nur in Anwesenheit einer Zielsequenz (eines spezifischen Liganden) stark fluoreszieren, können sie in Lösung verwendet werden, ohne dass nicht-hybridisierte Sonde entfernt werden muss. Molekulare Beacons sind hochspezifisch, so dass Fluoreszenz vollständig unterdrückt wird, wenn die Zielsequenz eine einzige falsche Base in der Oligonukleotidkette aufweist. Nachteilig an der Verwendung molekularer Beacons ist jedoch, dass sie aufgrund ihres Wirkmechanismus auf den Nachweis von Nukleinsäuren beschränkt sind. Sie können nicht für den Nachweis von anderen Rezeptor-Liganden-Komplexen eingesetzt werden.

Zur Bestimmung anderer Rezeptor-Liganden-Komplexe, insbesondere zum Nachweis von Antigen-Antikörperreaktionen werden bisher Biosensoren verwendet, auf denen Rezeptoren immobilisiert sind. Die Menge an gebundenem Rezeptor kann bisher nur unzureichend bestimmt werden. Die Mengenbestimmung beruht in der Regel auf der Messung, wie viel Flüssigkeit beim Druckprozess des Sensors abgegeben wird. Außerdem kann mit Hilfe verschiedener bekannter Färbetechniken bei einzelnen gedruckten Sensoren stichprobenhaft die Rezeptordichte ermittelt werden. Als besonders nachteilig wird dabei empfunden, dass zum Zeitpunkt der Messung der Interaktion zwischen Rezeptor und Ligand keine Aussage über die Menge des immobilisierten Rezeptors auf der Oberfläche des Biosensors möglich ist. Außerdem erlauben herkömmliche Biosensoren keine Messung der Rezeptordichte auf jedem individuellen Sensor und an jedem Messpunkt.

Der vorliegenden Erfindung liegt daher die Aufgabe zugrunde, ein verbessertes Verfahren zur Bestimmung der Zahl von Rezeptoren auf einer Trägeroberfläche vorzuschlagen, bei dem die Menge des tatsächlich immobilisierten Rezeptors exakt bestimmt werden kann. Darüber hinaus soll der Nachweis eines gebildeten Rezeptor-Liganden-Komplexes spezifisch erfolgen und nicht durch die Wahl des Markers beeinflusst werden.

Diese Aufgabe wird durch ein Verfahren zur Bestimmung der Zahl von Rezeptoren auf einem Träger gelöst, bei dem die Rezeptor-Marker-Komplexe unabhängig von den Rezeptor-Liganden-Komplexen nachgewiesen werden. In dem Verfahren wird zunächst ein Träger bereitgestellt. Auf dem Träger wird wenigstens ein Rezeptor immobilisiert, wobei der Rezeptor die Fähigkeit besitzt mit einem Liganden zu interagieren und einen spezifischen Rezeptor-Liganden-Komplex zu bildern.

Unter "Immobilisieren" wird jede dauerhafte Verbindung des Rezeptors mit der Oberfläche bzw. der Struktur des Trägers verstanden. Diese Interaktion kann beispielsweise auf wenigstens einer kovalenten Bindung oder wenigstens einer Disulfidbrücke beruhen. Darüber hinaus sind auch lösbare Verbindungen zwischen Rezeptor und Trägeroberfläche denkbar und geeignet, wobei ionische Wechselwirkungen vorteilhaft sind, die einfach durch pH-Wert Veränderungen gelöst werden können. Unter "Rezeptor-Liganden-Komplex" wird jede Art von Verbindung bzw. Interaktion von Rezeptor und Ligand verstanden. Somit ist der Begriff "Rezeptor-Liganden-Komplex" nicht auf die chemische Definition des Begriffs "Komplex" beschränkt.

Anschließend wird ein Signalmolekül bzw. ein Marker mit dem Rezeptor in Kontakt gebracht, wodurch sich ein Rezeptor-Marker-Komplex bildet, Danach wird die Zahl der Rezeptoren auf dem Träger ermittelt, indem die Rezeptor-Marker-Komplexe nachgewiesen werden.

Dann wird die Verbindung zwischen dem Marker und dem Rezeptor gelöst, um danach einen Rezeptor-Liganden-Komplexen bilden zu können. Das Lösen der Verbindung zwischen dem Marker und dem Rezeptor kann beispielsweise durch die Änderung der Temperatur, des pH-Wertes, der Ionenkonzentration oder des Salzgehaltes des umgebenden Milieus erfolgen.

Dadurch, dass die Rezeptor-Marker-Komplexe unabhängig von den Rezeptor-Liganden-Komplexen nachgewiesen werden, kann die Konzentration des Rezeptors direkt bestimmt werden. Da für gewöhnlich die Bindungskonstante, d.h. die Affinität des Liganden zu seinem Rezeptor bekannt ist, kann über die Rezeptorkonzentration und die Bindungskonstante die Konzentration des Liganden in einer zu analysierenden Probe berechnet werden. Darüber hinaus kann mit Hilfe dieses Verfahrens der Herstellungsprozess von Biosensoren überwacht werden, weil fehlerhaft gedruckte oder immobilisierte Sensoren einfach erkannt und aussortiert werden können.

Das Immobilisieren des Rezeptors auf dem Träger und das in Kontakt bringen des Markers mit dem Rezeptor kann auch gleichzeitig in einem einzigen Schritt erfolgen. So kann das

Verfahren besonders einfach und schnell durchgeführt werden, was insbesondere bei diagnostischen Routineuntersuchungen und sogenannten Schnelltests, die innerhalb kürzester Zeit ein zuverlässiges Ergebnis liefern müssen, von besonderer Bedeutung ist.

Zusätzlich zu den oben genannten Verfahrensschritten kann der Rezeptor mit einer Testprobe in Kontakt gebracht werden, die auf ihren Gehalt an Liganden untersucht werden soll. Die Inkubation des Rezeptors mit der Testprobe kann nach dem Immobilisieren des Rezeptors auf dem Träger, aber auch nach dem in Kontakt bringen des Markers mit dem Rezeptor oder nach der Ermittlung der Zahl der Rezeptoren auf dem Träger erfolgen.

Falls der Rezeptor mit einer Testprobe in Kontakt gebracht wird, die auf ihren Gehalt an Liganden untersucht werden soll, ist es vorteilhaft, die gebildeten Rezeptor-Liganden-Komplexe direkt und unabhängig von den gebildeten Rezeptor-Marker-Komplexen nachzuweisen.

Der Träger kann ein Halbleiter sein. Seine Oberfläche kann aus Silizium bzw. Halbmetalloxiden bestehen. Besonders vorteilhaft sind dabei SiOₓ oder Aluminiumoxid.

Der im Rahmen der Erfindung verwendete Rezeptor kann jedes Molekül mit einer Bindungsaffinität für einen bestimmten Liganden sein. Rezeptoren können natürlich vorkommend oder künstlich erzeugt sein. Sie können ebenso in ihrem natürlichen Zustand oder als Aggregate mit anderen Molekülen vorliegen. Rezeptoren binden direkt oder indirekt über spezifische Bindungssubstanzen oder Bindungsmoleküle kovalent oder nicht kovalent an den Liganden. Beispiele für Rezeptoren sind Enzyme, Antikörper, insbesondere monoklonale oder polyklonale Antikörper sowie funktionellen Fragmenten davon, Antiseren, Proteine, Oligo- und Polypeptide, Zellmembranrezeptoren, Nukleinsäuren, insbesondere DNA, RNA, cDNA, PNA, Oligo- und Polynukleotide, Zuckerbestandteile wie Saccharide, insbesondere Mono-, Di-, Tri-, Oligo- und Polysaccharide sowie Lecithin, Kofaktoren, zelluläre Membrane, Organelle, sowie Lipide und deren Derivate.

Wesentlich ist, dass Rezeptoren mit den korrespondierenden Liganden durch ihre molekulare Erkennung einen Rezeptor-Liganden-Komplex bilden. Demzufolge sind Liganden Moleküle, die durch einen bestimmten Rezeptor erkannt werden. Auch sie können natürlich vorkommen oder künstlich erzeugt sein. Beispiele für bekannte Liganden sind Agonisten und Antagonisten zelluläre Membranrezeptoren, Toxine, virale und bakterielle Epitope, insbesondere Antigene, Hormone (Opiate, Steroide, etc.), Peptide, Enzyme, Enzymsubstrate und Kofaktoren.

Obwohl die Bindung zwischen Rezeptor und Ligand im Rezeptor-Liganden-Komplex hochspezifisch ist, kann sie dennoch beispielsweise durch die Änderung der Temperatur, des pH-Wertes, der Ionenkonzentration, des Salzgehaltes des umgebenden Milieus oder der Anwesenheit von konkurrierenden Molekülen, lösbar sein.

Falls das Fluorochrom des Liganden eine größere Fluoreszenzlebensdauer als das Fluorochrom des Markers besitzt, können die Marker hochselektiv voneinander unterschieden werden. Eine ähnliche Wirkung kann durch Verwendung von Farbstoffen mit unterschiedlichen Anregungs- und Emissionsspektren erzielt werden. Falls der Ligand und der Marker an dieselbe Stelle des Rezeptors binden und somit um diese Bindung miteinander konkurrieren (sogenannter kompetitiver Antagonismus), ist es vorteilhaft wenn der Marker eine geringere Bindungsaffinität zu dem Rezeptor aufweist.

Die Markierung der Rezeptoren mit Markern erfolgt statistisch, d.h. dass nicht jeder einzelne Rezeptor individuell markiert sein muss. Dennoch befinden sich durchschnittlich auf n-Rezeptoren n-Marker. Darüber hinaus kann die Markierung auch ein Vielfaches von n betragen. Wesentlich dabei ist, dass die Marker nicht mit dem Prinzip der Messung interferieren.

Die Marker können reaktive Gruppe aufweisen, wobei insbesondere chemisch reaktive Gruppen mit hoher Spezifität wie z.B. Thiolgruppen als reaktive Gruppen geeignet sind. Durch solche chemisch reaktive Gruppen des Markers wird das Bindungsverhalten des Liganden an den Rezeptor nicht nennenswert beeinträchtigt.

Der Marker kann ein Farbstoff, insbesondere ein Lumineszenz-Farbstoff, vor allem ein Chemolumineszenz-, Photolumineszenz- oder Biolumineszenz-Farbstoff sein.

Wenn der Marker ein Fluoreszenz-Farbstoff ist, dann kann er ein Fluorochrom aufweisen. Hier sind insbesondere Rhodamin, vor allem Tetramethylrhodaminisothiocyanat (= TRITC) besonders geeignet. Solche Fluorochrome können als Maleinimide zur Konjugation verwendet werden. Wenn der Rezeptor ein Antikörper ist, kann dieser mit reaktiven Farbstoffen konjugiert werden. Eine Reihe von Beispielen können hierzu aus der Publikation von G. T. Herrmannson "Bioconjugate techniques", A-cademic Press 1996, entnommen werden.

Darüber hinaus können als Rezeptoren sogenannte chimäre Proteine, die künstlich aus Proteinbestandteilen unterschiedlichen, z.B. biologischen und künstlichen Ursprungs zusammengesetzt sind, verwendet werden. So kann beispielsweise ein Antikörper, bei dem die konstante Region (Fab-Region) durch ein fluoreszierendes Protein ersetzt wurde, so dass nur die variablen Regionen zur Antigenerkennung erhalten bleiben, als Rezeptor verwendet werden. Das fluoreszierende Protein kann insbesondere ein green fluorescent protein (GFP) oder ein blue fluorescent protein (BFP) sein.

Der Rezeptor kann darüber hinaus eine Eigenfluoreszenz aufweisen. Eine solche Eigenfluoreszenz ist insbesondere von der natürlich vorkommenden Aminosäure Tryptophan bekannt, die in nahezu allen größeren Proteinen vorkommt. Wenn demnach der Rezeptor ein Antikörper, Protein oder Oligopeptid ist, in dem mindestens ein Tryptophan vorkommt, kann die Eigenfluoreszenz dieser Aminosäure für den Nachweis verwendet werden.

Die Bindung zwischen Rezeptor und Marker in dem Rezeptor-Marker-Komplex weist eine Fluoreszenzhalbwertszeit im Bereich von Nanosekunden auf.

Der Rezeptor-Marker-Komplex kann einen "fluorescence resonance energy transfer" (= FRET) aufweisen. Bei diesem System kommt es zu einem Energietransfer zwischen einem Donor, der die Energie abgibt und einem Akzeptor, der die Energie aufnimmt.

Die Fluoreszenz des FRET kann durch die Interaktion des Liganden mit dem Rezeptor verändert werden. Der Donor und der Akzeptor des FRET können auf dem Rezeptor immobilisiert sein. Durch Bindung des Liganden an den Rezeptor werden der Donor und der Akzeptor räumlich voneinander getrennt, so dass eine Fluoreszenzauslöschung beim Akzeptor erfolgt, wobei der Akzeptor ein Fluorochrom ist. Andererseits kann auch eine Fluoreszenzentstehung beim Donor erfolgen, wobei der Akzeptor als Fluoreszenzquencher bezeichnet wird. Darüber hinaus kann der Ligand selbst als Donor wirken, so dass er entweder fluoresziert oder quencht. Außerdem ist denkbar, dass der Ligand durch seine Bindung an den Rezeptor unter Ausbildung des Rezeptor-Liganden-Komplexes den FRET-Donor und -Akzeptor in unmittelbaren direkten Kontakt zueinander bringt.

Darüber hinaus kann FRET sowohl mit einem Quencher als auch einem Fluorochrom als Akzeptor funktionieren. Ist der Akzeptor ein Quencher, so wird die Fluoreszenz ausgelöscht. Ist der Akzeptor ein Fluorochrom, so wird die Energie des Donors von dem Akzeptor als Fluoreszenzlicht wieder abgegeben.

Ebenso können Liganden verwendet werden, die selbst fluoreszenzmarkiert sind. Auf diese Weise wird unter Verwendung eines ebenfalls markierten Kompetitors, der beispielsweise ein fluoreszenzmarkierter Ligand sein kann, ein kompetitiver Assay möglich. Der Kompetitor erzeugt (oder löscht) ein Fluoreszenzsignal auf dem Rezeptor. Dabei ist vor allem das Erzeugen eines Signals vorteilhaft, weil eine unspezifische Bindung des Liganden/des Kompetitors an Bereiche der Oberfläche außerhalb des Rezeptors nicht signalbildend ist.

Der Marker kann jede beliebige nachweisbare Form annehmen, wobei der Marker insbesondere ein Mikropartikel sein kann.

## Patentansprüche

1. Verfahren zur Bestimmung der Zahl von Rezeptoren auf einem Träger, wobei das Verfahren die Schritte umfasst:
(a) Bereitstellen eines Trägers;
(b) Immobilisieren wenigstens eines Rezeptors auf dem Träger, wobei der Rezeptor die Fähigkeit besitzt mit einem Liganden zu interagieren und einen Rezeptor-Liganden-Komplex zu bilden;
(c) nach Immobilisieren des wenigstens einen Rezeptors auf dem Träger: In Kontakt bringen eines Markers mit dem Rezeptor, um einen Rezeptor-Marker-Komplex mit einer lösbaren Bindung zwischen Rezeptor und Marker zu bilden;
(d) Ermitteln der Zahl der Rezeptoren auf dem Träger indem die Rezeptor-Marker-Komplexe nachgewiesen werden;
wobei die Rezeptor-Marker-Komplexe unabhängig von Rezeptor-Liganden-Komplexen nachgewiesen werden.

2. verfahren nach Anspruch 1, **dadurch gekennzeichnet, dass** nach Schritt b) oder c) oder d) zusätzlich der schritt (i) durchgeführt wird:
(i) In Kontakt bringen des Rezeptors mit einer Testprobe, die auf ihren Gehalt an Liganden untersucht wird.

3. Verfahren nach Anspruch 2, **dadurch gekennzeichnet, dass** nach Schritt (i) zusätzlich der Schritt (ii) durchgeführt wird:
(ii) Nachweisen der Rezeptor-Liganden-Komplexe.

4. verfahren nach einem der vorhergehenden Ansprüche, **dadurch gekennzeichnet, dass** der Träger ein Halbleiter mit einer oberfläche aus Silizium, Halbmetalloxiden, insbesondere SiOₓ oder Aluminiumoxid ist.

5. Verfahren nach einem der vorhergehenden Ansprüche, **dadurch gekennzeichnet, dass** der Rezeptor ausgewählt ist aus der Gruppe bestehend aus Antikörpern, insbesondere monoklonalen oder polyklonalen Antikörpern sowie funktionellen Fragmenten davon; Proteinen, Oligo- und Polypeptiden, Nukleinsäure, insbesondere DNA, RNA, cDNA, PNA, Oligo- und Polynukleotiden; sowie Sacchariden, insbesondere Mono-, Di-, Tri-, Oligo- und Polysacchariden.

6. Verfahren nach einem der vorhergehenden Ansprüche, **dadurch gekennzeichnet, dass** die Bindung zwischen Rezeptor und Ligand in dem Rezeptor-Liganden-Komplex lösbar ist.

7. Verfahren nach einem der vorhergehenden Ansprüche, **dadurch gekennzeichnet, dass** die Bindung zwischen Rezeptor und Ligand eine Halbwertzeit im Bereich von Mikrosekunden (= µs) oder größer aufweist.

8. Verfahren nach einem der vorhergehenden Ansprüche, **dadurch gekennzeichnet, dass** n-Rezeptoren n-Marker oder ein Vielfaches von n an Markern zugeordnet sind.

9. Verfahren nach einem der vorhergehenden Ansprüche, **dadurch gekennzeichnet, dass** der Marker reaktive Gruppen, insbesondere Thiolgruppen aufweiset.

10. Verfahren nach einem der vorhergehenden Ansprüche, **dadurch gekennzeichnet, dass** der Marker ein Farbstoff, insbesondere ein Lumineszenz-Farbstoff, vor allem ein Chemolumineszenz-, Photolumineszenz- oder Biolumines-zenz-Farbstoff ist.

11. Verfahren nach einem der vorhergehenden Ansprüche, **dadurch gekennzeichnet, dass** der Marker ein Fluoreszenz-Farbstoff, vorzugsweise ein Fluorochrom, weiter bevorzugt ein Rhodamin, vor allem Tetramethylrhodaminisothiocyanat (= TRITC) ist.

12. Verfahren nach einem der vorhergehenden Ansprüche, **dadurch gekennzeichnet, dass** der Rezeptor eine Eigenfluoreszenz aufweist.

13. Verfahren nach Anspruch 12, **dadurch gekennzeichnet, dass** die Aminosäure Tryptophan die Eigenfluoreszenz liefert.

14. verfahren nach einem der vorhergehenden Ansprüche, **dadurch gekennzeichnet, dass** die Bindung zwischen Rezeptor und Marker eine Fluoreszenzhalbwertszeit im Bereich von Nanosekunden (= ns) aufweist.

15. Verfahren nach einem der vorhergehenden Ansprüche, **dadurch gekennzeichnet, dass** der Rezeptor-Marker-Komplex einen "fluorescence resonance energy transfer" (= FRET) aufweist.

16. verfahren nach Anspruch 15, **dadurch gekennzeichnet, dass** die Fluoreszenz des FRET durch die Interaktion des Liganden mit dem Rezeptor verändert wird.

17. Verfahren nach Anspruch 15 oder 16, **dadurch gekennzeichnet, dass** der Rezeptor den Donor und den Akzeptor des FRET aufweist.

18. Verfahren nach einem der Ansprüche 15 bis 17, **dadurch gekennzeichnet, dass** die Fluoreszenz beim Donor entsteht oder die Fluoreszenz beim Akzeptor ausgelöscht wird.

19. Verfahren nach einem der Ansprüche 15 bis 18, **dadurch gekennzeichnet, dass** der Ligand als Donor des FRET wirt.

20. Verfahren nach einem der Ansprüche 15 bis 18, **dadurch gekennzeichnet, dass** der Ligand den Donor und den Akzeptor des FRET direkt in Kontakt bringt.

21. Verfahren nach einem der vorhergehenden Ansprüche, **dadurch gekennzeichnet, dass** fluoreszenzmarkierte Liganden verwendet werden.

22. Verfahren nach einem der vorhergehenden Ansprüche, **dadurch gekennzeichnet, dass** der Marker ein Mikropartikel ist.

## Claims

1. Procedure for determining the number of receptors on a carrier, said procedure comprising the following steps:
(a) Preparation of a carrier;
(b) Immobilization of at least one receptor on the carrier, wherein the receptor has the ability to interact with a ligand and form a receptor-ligand complex;
(c) Following the immobilization of the one receptor at least on the carrier: a marker is brought into contact with the receptor to create a receptor-marker complex with a non-permanent bond between the receptor and the marker;
(d) Determination of the number of receptors on the carrier by establishing the receptor-marker complexes;
wherein the receptor-marker complexes are established independently of the receptor-ligand complexes.

2. Procedure as claimed in Claim 1, **characterized in that** after step (b) or (c) or (d), step (i) is also performed:
(i) The receptor is brought into contact with a test sample whose ligand content is examined.

3. Procedure as claimed in Claim 2, **characterized in that** after step (i), step (ii) is also performed:
(ii) Establishment of the receptor-ligand complexes.

4. Procedure as claimed in one of the previous claims, **characterized in that** the carrier is a semi-conductor with a surface made of silicium, metalloid oxides, notably SiOₓ or aluminum oxide.

5. Procedure as claimed in one of the previous claims, **characterized in that** the receptor is selected from the group of existing antibodies, especially monoclonal or polyclonal antibodies as well as functional fragments of same; proteins, oligopeptides and polypeptides, nucleic acids, notably DNA, RNA, cDNA, PNA, oligo-nucleotides, polynucleotides; as well as saccharides, notably monosaccharides, disaccharides, trisaccharides, oligosaccharides and polysaccharides.

6. Procedure as claimed in one of the previous claims, **characterized in that** the bond between the receptor and ligand in the receptor-ligand complex is non-permanent.

7. Procedure as claimed in one of the previous claims, **characterized in that** the bond between the receptor and the ligand has a half-life in the region of mircoseconds (µs) or higher.

8. Procedure as claimed in one of the previous claims, **characterized in that** n markers or a multiple of n markers are assigned to the n receptors.

9. Procedure as claimed in one of the previous claims, **characterized in that** the marker has reactive groups, particularly thiol groups.

10. Procedure as claimed in one of the previous claims, **characterized in that** the marker is a dye, particularly a luminescent dye, especially a chemiluminescent, photoluminescent or bioluminescent dye.

11. Procedure as claimed in one of the previous claims, **characterized in that** the marker is a fluorescent dye, preferably a fluorochrome, more preferably a rhodamine, especially TRITC (Tetramethyl Rhodamine Iso-Thiocyanate).

12. Procedure as claimed in one of the previous claims, **characterized in that** the receptor exhibits intrinsic fluorescence.

13. Procedure as claimed in Claim 12, **characterized in that** tryptophan - an amino acid - provides the intrinsic fluorescence.

14. Procedure as claimed in one of the previous claims, **characterized in that** the bond between the receptor and the marker has a fluorescence half-life in the nanosecond (ns) region.

15. Procedure as claimed in one of the previous claims, **characterized in that** the receptor-marker complex demonstrates a fluorescence resonance energy transfer (FRET).

16. Procedure as claimed in Claim 15, **characterized in that** the fluorescence of the FRET is changed by the interaction of the ligand with the receptor.

17. Procedure as claimed in Claim 15 or 16, **characterized in that** the receptor has the donor and the acceptor of the FRET.

18. Procedure as claimed in one of the Claims 15 to 17, **characterized in that** the fluorescence originates with the donor and the fluorescence is extinguished with the acceptor.

19. Procedure as claimed in one of the Claims 15 to 18, **characterized in that** the ligand acts as the FRET donor.

20. Procedure as claimed in one of the Claims 15 to 18, **characterized in that** the ligand brings the donor and acceptor of the FRET into contact.

21. Procedure as claimed in one of the previous claims, **characterized in that** ligands marked by fluorescence are used.

22. Procedure as claimed in one of the previous claims, **characterized in that** the marker is a microparticle.

## Revendications

1. Procédé destiné à la détermination du nombre de récepteurs sur un support, le procédé comprenant les étapes suivantes :
(a) Préparation d'un support ;
(b) Immobilisation d'au moins un récepteur sur le support, le récepteur possédant la faculté à interagir avec un ligand et à former un complexe ligand-récepteur ;
(c) Après immobilisation de l'au moins un récepteur sur le support : mise en contact d'un marqueur avec le récepteur, afin de former un complexe marqueur-récepteur avec une liaison amovible entre le récepteur et le marqueur ;
(d) Détermination du nombre de récepteurs sur le support en ce que les complexes marqueur-récepteur sont détectés ;
les complexes marqueur-récepteur étant détectés indépendamment des complexes ligand-récepteur.

2. Procédé selon la revendication 1, **caractérisé en ce qu'**après l'étape (b) ou (c) ou (d) est exécutée en plus l'étape (i) :
(i) Mise en contact du récepteur avec un échantillon de test, lequel est analysé par rapport à la teneur en ligands.

3. Procédé selon la revendication 2, **caractérisé en ce qu'**après l'étape (i) est exécutée en plus l'étape (ii) :
(ii) Détection des complexes ligand-récepteur.

4. Procédé selon l'une des revendications précédentes, **caractérisé en ce que** le support est un semi-conducteur avec une surface en silicium, des oxydes métalloïdes, notamment SiOₓ ou un oxyde d'aluminium.

5. Procédé selon l'une des revendications précédentes, **caractérisé en ce que** le récepteur est sélectionné à partir du groupe composé d'anticorps, notamment des anticorps monoclonaux ou polyclonaux, ainsi que de fragments fonctionnels d'entre eux ; protéines, oligopeptides et polypeptides, acides nucléiques, notamment ADN, ARN, cADN, ANP, oligonucléotides, polynucléotides ; ainsi que saccharides, notamment les mono-, di-, tri-, oligo- et polysaccharides.

6. Procédé selon l'une des revendications précédentes, **caractérisé en ce que** la liaison entre le récepteur et le ligand est amovible dans le complexe ligand-récepteur.

7. Procédé selon l'une des revendications précédentes, **caractérisé en ce que** la liaison entre le récepteur et le ligand présente une demi-vie se situant dans la plage de la microseconde (µs).

8. Procédé selon l'une des revendications précédentes, **caractérisé en ce qu'**aux n récepteurs sont attribués n marqueurs ou un multiple de n de marqueurs.

9. Procédé selon l'une des revendications précédentes, **caractérisé en ce que** le marqueur comporte des groupes réactifs, notamment des groupes de thiol.

10. Procédé selon l'une des revendications précédentes, **caractérisé en ce que** le marqueur est un colorant, notamment un colorant luminescent, surtout un colorant chimioluminescent, photoluminescent ou bioluminescent.

11. Procédé selon l'une des revendications précédentes, **caractérisé en ce que** le marqueur est un colorant fluorescent, de préférence un fluorochrome, particulièrement de préférence une rhodamine, surtout le TRITC (Tetra methyl Rhodamine Iso Thio Cyanate).

12. Procédé selon l'une des revendications précédentes, **caractérisé en ce que** le récepteur présente une autofluorescence.

13. Procédé selon la revendication 12, **caractérisé en ce que** le tryptophane - un acide aminé - fournit l'autofluorescence.

14. Procédé selon l'une des revendications précédentes, **caractérisé en ce que** la liaison entre le récepteur et le marqueur présente une demi-vie de fluorescence se situant dans la plage de la nanoseconde (ns).

15. Procédé selon l'une des revendications précédentes, **caractérisé en ce que** le complexe marqueur-récepteur présente un FRET ("fluorescence resonance energy transfer" = "transfert d'énergie par résonance en fluorescence).

16. Procédé selon la revendication 15, **caractérisé en ce que** la fluorescence du FRET est modifiée par l'interaction du ligand avec le récepteur.

17. Procédé selon la revendication 15 ou 16, **caractérisé en ce que** le récepteur comprend le donneur et l'accepteur du FRET.

18. Procédé selon la revendication 15 à 17, **caractérisé en ce que** la fluorescence est activée pour le donneur ou la fluorescence est éteinte pour l'accepteur.

19. Procédé selon la revendication 15 à 18, **caractérisé en ce que** le ligand agit en tant que donneur du FRET.

20. Procédé selon la revendication 15 à 18, **caractérisé en ce que** le ligand met en contact direct le donneur et l'accepteur du FRET.

21. Procédé selon l'une des revendications précédentes, **caractérisé en ce que** des ligands marqués par fluorescence sont utilisés.

22. Procédé selon l'une des revendications précédentes, **caractérisé en ce que** le marqueur est une microparticule.
